(19) Europäisches Patentamt
European Patent Office
Office européen des brevets

(11) **EP 4 079 274 B1**

(12) **EUROPEAN PATENT SPECIFICATION**

(45) Date of publication and mention
of the grant of the patent:
**21.02.2024 Bulletin 2024/08**

(51) International Patent Classification (IPC):
**A61F 13/42** (2006.01)    **A61F 13/531** (2006.01)
**A61F 13/49** (2006.01)

(21) Application number: **21169677.8**

(22) Date of filing: **21.04.2021**

(52) Cooperative Patent Classification (CPC):
**A61F 13/42; A61F 13/49001; A61F 13/531**

(54) **ABSORBENT ARTICLES HAVING A WETNESS INDICATOR**

ABSORBIERENDER ARTIKEL MIT NÄSSEANZEIGER

ARTICLES ABSORBANTS DOTÉS D'UN INDICATEUR D'HUMIDITÉ

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**26.10.2022 Bulletin 2022/43**

(73) Proprietors:
• **Ontex BV**
**9255 Buggenhout (BE)**
• **Ontex Group NV**
**9320 Erembodegem (BE)**

(72) Inventors:
• **Ingenfeld, Björn**
**53115 Bonn (DE)**
• **Saheb, Mohammed Moula**
**56727 Mayen (DE)**
• **Lambertz, Christina**
**56566 Neuwied (DE)**
• **Weber, Ainas**
**53474 Bad Neuenahr-Ahrweiler (DE)**
• **Cobbaert, Dries**
**1770 Liedekerke (BE)**

(74) Representative: **Larangé, Françoise**
**Ontex BVBA
Korte Keppestraat 21
9320 Aalst (BE)**

(56) References cited:
EP-A1- 3 620 144      WO-A1-2019/158226
US-A1- 2017 079 857    US-B2- 9 789 009
US-B2- 10 543 129

## Description

### TECHNICAL FIELD

The present disclosure pertains to the technical field of absorbent hygiene products that can be used as articles for absorbing body fluids and exudates, such as urine and fecal material, or blood, menses, and vaginal fluids. In particular, the present disclosure relates to absorbent garments (or articles), such as disposable diapers or diaper pants, disposable incontinence diapers or pants, which are configured to collect and contain urine and fecal material and avoid leakage, or sanitary napkins or panty liners, which are configured to collect and contain blood, menses, urine, vaginal fluids and avoid leakage. More particularly the present disclosure relates to such products having a wetness indicator which can indicate the presence of a liquid bodily exudate in the article.

### BACKGROUND

Absorbent cores have been subject to considerable improvement and innovation over time to address needs such as improved fluid absorption and distribution, as well as comfort, and a need for continued improvement exists. Such needs are ever present in today's demanding consumer environment.

In this context, more recently, absorbent cores having one or more channels substantially free of absorbent material have been developed, as described for example in EP3342386A1 or in European patent application 20171374.0, disclosing channels beneficial in terms of fluid handling. Channeled core particularly designed to provide exceptionally fast absorption and good liquid distribution throughout the entire core are thus available, leading to improved absorption capacity and low rewet that provides to the consumer an enhanced perception of dryness. They further may offer an improved fit to the wearer, thereby providing comfort and minimizing the risk of leakage.

In parallel, signals to the caregivers for changing the article when it becomes full are known and are for example described in WO2010/078304 A1 but not in combination with channels, or in WO2015/095514 A2 or WO2019/125230 A1 or US2017079857 in the context of a pair of generally longitudinally-extending channels symmetrically disposed relative to the longitudinal axis.

WO2010/078304 A1 describes a system acting as a fullness indicator which can help provide certainty about the fullness of the absorbent article, comprising a primary visual fullness indicator and a secondary visual wetness indicator. Each indicator comprises a series or pattern of indicating areas, which makes the construction and method of manufacturing such absorbent article complex, with intermittent application of wetness indicating material at different registered locations.

WO2015/095514 A2 and WO2019/125230 A1 describe absorbent articles having a pair of generally longitudinally-extending channels symmetrically disposed relative to the longitudinal axis wherein the wetness indicator is placed, when seen from the exterior of the article, in one case between the two channel-forming areas and/or between any of the channel-forming areas and any of the longitudinally extending side edges of the article and in the other case, only in the front and/or back portions out of the channels.

WO2016/196069 A1 describes absorbent articles having a pair of generally longitudinally-extending channels symmetrically disposed relative to the longitudinal axis wherein the wetness indicator is placed, when seen from the exterior of the article, superposed with at least one these channels. This is said to react quickly to a first liquid insult, providing a very early warning to the caregiver that urination has occurred. However this type of signal could be misunderstood and encourage the caregiver to change the article too early.

There is thus still a need to improve absorbent articles comprising both a channelled core and a wetness indicator to know how full the article is, so that the article can be changed after the wearer has appropriately utilized the capacity of the article and before it is likely to leak. This need is particularly true for articles where the channel includes a substantial portion coincident with the longitudinal center line, i.e. along which the miction occurs, so that the wetness indicator does not give a too early message that the article should be changed. Furthermore there is a need to manufacture such improved absorbent articles in a simple way, avoiding complex positioning and/or complex intermittent distribution of the wetness indicator material.

### SUMMARY

In one aspect, the present disclosure relates to an absorbent article comprising a liquid permeable topsheet, a liquid impermeable backsheet and an absorbent core positioned in between said topsheet and said backsheet. The absorbent core has a first and second longitudinal edge and a front and back transverse edge. It has a longitudinal center line dividing the absorbent core in a first longitudinal portion and a second longitudinal portion on either side of the longitudinal center line. It also has a transverse center line dividing the absorbent core in a front portion and a back portion on either side of the transverse center line. The longitudinal center line and the transverse center line together create four quadrants within the absorbent core. The absorbent core comprises absorbent material selected from the group consisting of cellulose fibers, superabsorbent polymers and combinations thereof, and the absorbent material is contained within at least one core wrap substrate enclosing said absorbent material. The top layer of said core wrap is adhered to a bottom layer of said core wrap to form one or more channels substantially free of said absorbent material. At least one channel follows a substantially continuous path from any point of said channel to any other point of the same channel and comprises at least one longitudinally extending channel central section coincident with the longitudinal center line and closest to the transverse center line. The absorbent article further comprises a continuously extending wetness indicator, which as seen from the garment-facing side of the article, is coincident with the longitudinal center line. Said wetness indicator comprises an overlapping portion superposed with at least part of the longitudinally extending channel central section and an exposed portion superposed with absorbent material at least partially surrounded by channel sections closer to the front transverse edge.

We have found that such a combination of channel and wetness indicator offers an easy-to-manufacture absorbent article able to indicate a sequence of wetting events and to give an accurate indication of how full the article is.

The overlapping portion of the wetness indicator superposed with at least part of the longitudinally extending channel central section may offer a quick response to a first wetness event, which for example might be helpful to be noticed in case of a health issue, whilst the exposed portion superposed with absorbent material at least partially surrounded by channel sections closer to the front transverse edge might indicate the approaching fullness of the diaper. In addition, the fact that the wetness indicator is a centrally positioned continuously extending element in machine direction makes it easy to produce.

Other objects and advantages of this invention will become apparent hereinafter.

## BRIEF DESCRIPTION OF THE FIGURES

Fig. 1 to 14 show diagrammatic top views of absorbent cores according to embodiments herein.

Fig. 15 shows a side view of the bended apparatus as used in the example tests.

## DETAILED DESCRIPTION

Unless otherwise defined, all terms used in disclosing characteristics of the disclosure, including technical and scientific terms, have the meaning as commonly understood by one of ordinary skill in the art to which this disclosure belongs. By means of further guidance, term definitions are included to better appreciate the teaching of the present disclosure.

As used herein, the following terms have the following meanings:

"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1 % or less of and from the specified value, in so far such variations are appropriate to perform in the disclosed disclosure. However, it is to be understood that the value to which the modifier "about" refers is itself also specifically disclosed.

"Comprise", "comprising", and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

The expression "% by weight" (weight percent), here and throughout the description unless otherwise defined, refers to the relative weight of the respective component based on the overall weight of the formulation.

The recitation of numerical ranges by endpoints includes all numbers and fractions subsumed within that range, as well as the recited endpoints.

"Absorbent article" refers to devices that absorb and contain liquid, and more specifically, refers to devices that are placed against or in proximity to the body of the wearer to absorb and contain the various exudates discharged from the body. Absorbent articles include but are not limited to diapers, adult incontinence briefs, training pants, diaper holders and liners, sanitary napkins and the like, as well as surgical bandages and sponges. Absorbent articles preferably comprise a longitudinal axis and a transversal axis perpendicular to said longitudinal axis. The longitudinal axis is hereby conventionally chosen in the front-to-back direction of the article when referring to the article being worn, and the transversal axis is conventionally chosen in the left-to-right direction of the article when referring to the article being worn. Disposable absorbent articles can include a liquid pervious top sheet, a back sheet joined to the top sheet, and an absorbent core positioned and held between the top sheet and the back sheet. The top sheet is operatively permeable to the liquids that are intended to be held or stored by the absorbent article, and the back sheet may or may not be substantially impervious or otherwise operatively impermeable to the intended liquids. The absorbent article may also include other components, such as liquid wicking layers, liquid intake layers, liquid distribution layers, transfer layers, barrier layers, wrapping layers and the like, as well as combinations thereof. Disposable absorbent articles and the components thereof can operate to provide a body-facing surface and a garment-facing surface.

An absorbent article, such as a diaper, comprises a front waistband region, a back waistband region, an intermediate crotch region which interconnects the front and rear waistband regions. When used herein, reference to a "front" portion refers to that part of the absorbent article which is generally located on the front of a subject, such as an infant or adult, when in use. Reference to the "rear" or "back" "portion" or "section" refers to the portion of the absorbent article generally located at the rear of the subject, such as an infant or adult, when in use, and reference to the "crotch" portion refers to that portion which is generally located between the legs of subject, such as an infant or adult, when in use. The crotch region is an area where repeated fluid surge typically occurs, within the absorbent article assembly.

"Front", "rear or back", and "crotch" portions of the absorbent core as used herein typically refer to portions of the absorbent core that are proximal to respective portions of the absorbent article. For example, the "front" portion of the core is that which is most proximal to the front of the subject when worn, the "rear or back" portion of the core is that which is most proximal to the rear or back of the subject when worn, and the "crotch" portion of the core is the middle portion of the absorbent core between the "front" and "rear or back" portions.

Preferably, a diaper comprises a liquid permeable "top sheet", a liquid impermeable "back sheet", and an "absorbent medium" disposed between the top sheet and the back sheet. The top sheet, back sheet and the absorbent medium could be made from any suitable material known to the person skilled in the art. The top sheet is generally located at or near the bodyside surface of the article, while the back sheet is generally located at or near the garment-side surface of the article. Optionally, the article may comprise one or more separate layers which are in addition to the back sheet and are interposed between the back sheet and the absorbent medium. Top sheet and back sheet are connected or otherwise associated together in an operable manner.

The "absorbent medium" or "absorbent core" or "absorbent body" is the absorbent structure disposed between the top sheet and the back sheet of the absorbent article in at least the crotch region of the absorbent article and is capable of absorbing and retaining liquid body exudates. The size and the absorbent capacity of the absorbent medium should be compatible with the size of the intended wearer and the liquid loading imparted by the intended use of the absorbent article. Further, the size and the absorbent capacity of the absorbent medium can be varied to accommodate wearers ranging from infants through adults. It may be manufactured in a wide variety of shapes (for example, rectangular, trapezoidal, T-shape, I-shape, hourglass shape, etc.) and from a wide variety of materials. Examples of commonly occurring absorbent materials are cellulosic fluff pulp, tissue layers, highly absorbent polymers (so called superabsorbent polymer particles (SAP)), absorbent foam materials, absorbent nonwoven materials or the like. It is common to combine cellulosic fluff pulp with superabsorbent polymers in an absorbent material.

The "absorbent core length" is the length of the absorbent material taken along the longitudinal center line.

"Mechanical bond" is an attachment between two or more elements, components, regions, or webs and may comprise heat bonds, pressure bonds, ultrasonic bonds, dynamic mechanical bonds, or any other suitable non-adhesive attachment means or combinations of these attachment means as are known in the art.

"Acquisition and distribution layer", "ADL", "Acquisition and distribution system" or "surge management portion" refers to a sub-layer which preferably is a nonwoven wicking layer under the top sheet of an absorbent product, which speeds up the transport and improves distribution of fluids throughout the absorbent core. The surge management portion is typically less hydrophilic than the retention portion, and has the ability to quickly collect and temporarily hold liquid surges, and to transport the liquid from its initial entrance point to other parts of the absorbent structure, particularly the retention portion. This configuration can help prevent the liquid from pooling and collecting on the portion of the absorbent garment positioned against the wearer's skin, thereby reducing the feeling of wetness by the wearer. Preferably, the surge management portion is positioned between the top sheet and the retention portion.

The term "bulk density" as used herein refers to the weight of a material per unit of volume. Bulk density is generally expressed in units of weight/volume (e.g., grams per cubic centimeter). The bulk density of flat, generally planar materials such as, for example, fibrous nonwoven webs, may be derived from measurements of thickness and basis weight of a sample. The basis weight of the sample is determined essentially in accordance with ASTM D-3776-9 with the following changes: 1) sample size is cut to 10.16 cm X 10.16 cm (4 inches X 4 inches) square and 2); a total of 9 samples are weighed.

The term "adhesive" as used herein is intended to refer to any suitable hot melt, water or solvent borne adhesive that can be applied to a surface of a product component of the present disclosure. Accordingly, suitable adhesives include conventional hot melt adhesives, pressure-sensitive adhesives and reactive adhesives (i.e., polyurethane).

As used herein, the term "adhesive bonding" means a bonding process which forms a bond by application of an adhesive. Such application of adhesive may be by various processes such as slot coating, spray coating and other topical applications. Further, such adhesive may be applied within a product component and then exposed to pressure such that contact of a second product component with the adhesive containing product component forms an adhesive bond between the two components.

As used herein, an "airformed web" refers to a material comprising cellulosic fibers such as those from fluff pulp that have been separated, such as by a hammermilling process, and then deposited on a porous surface without a substantial quantity of binder fibers present. Airfelt materials used as the absorbent core in many diapers, for example, are a typical example of an airformed material.

As used herein, an "airlaid web" is a fibrous structure formed primarily by a process involving deposition of air-entrained fibers onto a mat, typically with binder fibers present, and typically followed by densification and thermal bonding. In addition to traditional thermally bonded airlaid structures (those formed with non-tacky binder material present and substantial thermally bonded), the scope of the term "airlaid" according to the present disclosure can also include coform, which is produced by combining air-entrained dry, dispersed cellulosic fibers with meltblown synthetic polymer fibers while the polymer fibers are still tacky. Further, an airformed web to which binder material is subsequently added can be considered within the scope of the term "airlaid" according to the present disclosure. Binder can be added to an airformed web in liquid form (e. g., an aqueous solution or a melt) by spray nozzles, direction injection or impregnation, vacuum drawing, foam impregnation, and so forth. Solid binder particles can also be added by mechanical or pneumatic means.

As used herein, an "air-through-bonded" nonwoven, is a nonwoven structure primarily formed by a process that comprises the application of heated air to the surface of the nonwoven fabric. During the through air bonding process, heated air flows through holes in a plenum above the nonwoven material. Unlike hot ovens, which push air through the material, the through air process uses negative pressure of suction to pull the air through an open conveyor apron holding nonwoven as it is drawn through the oven. Pulling air through the material allows the rapid and even transmission of heat to minimize distortion of the nonwoven material. The binding agents used in the through air bonding process include crystalline binder fibers and powders, which melt to form molten droplets throughout the cross-section of the nonwoven. As the material is cooled, bonding occurs at these droplet points.

As used therein, the term "associated" encompasses configurations in which top sheet is directly joined to back sheet by affixing top sheet directly to back sheet, and configurations wherein top sheet is joined to back sheet by affixing top sheet to intermediate members which in turn are affixed to back sheet. Top sheet and back sheet can be affixed directly to each other by attachment means such as an adhesive, sonic bonds, thermal bonds or any other attachment means known in the art. For example, a uniform continuous layer of adhesive, a patterned layer of adhesive, a sprayed pattern of adhesive or an array of separate lines, swirls or spots of construction adhesive may be used to affix top sheet to back sheet. It should be readily appreciated that the above-described attachment means may also be employed to interconnect and assemble together the various other component parts of the article described herein.

The term "back sheet" or "backsheet" refers to a material forming the outer cover of the absorbent article. The back sheet prevents the exudates contained in the absorbent structure from wetting articles such as bedsheets and overgarments which contact the disposable absorbent article. The back sheet may be a unitary layer of material or may be a composite layer composed of multiple components assembled side-by-side or laminated. The back sheet may be the same or different in different parts of the absorbent article. At least in the area of the absorbent medium the back sheet comprises a liquid impervious material in the form of a thin plastic film, e.g. a polyethylene or polypropylene film, a nonwoven material coated with a liquid impervious material, a hydrophobic nonwoven material, which resists liquid penetration, or a laminate of a plastic film and a nonwoven material. The back sheet material may be breathable so as to allow vapour to escape from the absorbent material, while still preventing liquids from passing there through. Examples of breathable back sheet materials are porous polymeric films, nonwoven laminates of spunbond and meltblown layers and laminates of porous polymeric films and nonwoven materials.

The term "blend" means a mixture of two or more polymers while the term "alloy" means a sub-class of blends wherein the components are immiscible but have been compatibilized.

As used herein, the "skin facing", "body-facing" or "bodyside" surface means that surface of the article or component which is intended to be disposed toward or placed adjacent to the body of the wearer during ordinary use, while the "outward", "outward-facing" or "garment-side" or "garment facing" surface is on the opposite side, and is intended to be disposed to face away from the wearer's body during ordinary use. Such outward surface may be arranged to face toward or placed adjacent to the wearer's undergarments when the absorbent article is worn.

"Bonded" refers to the joining, adhering, connecting, attaching, or the like, of at least two elements. Two elements will be considered to be bonded together when they are bonded directly to one another or indirectly to one another, such as when each is directly bonded to intermediate elements.

The term "breathable" refers to films having a water vapor transmission rate (WVTR) of at least 300 grams/m$^2$ - 24 hours.

"Carded web (or layer(s) or nonwoven)" refers to webs that are made from staple fibers that are sent through a combing or carding unit, which opens and aligns the staple fibers in the machine direction to form a generally machine direction-oriented fibrous nonwoven web. The web is then bonded by one or more of several known bonding methods. Bonding of nonwoven webs may be achieved by a number of methods; powder bonding, wherein a powdered adhesive or a binder is distributed through the web and then activated, usually by heating the web and adhesive with hot air; pattern bonding, wherein heated calendar rolls or ultrasonic bonding equipment are used to bond the fibers together, usually in a localized bond pattern, though the web can be bonded across its entire surface if so desired; through-air bonding, wherein air which is sufficiently hot to soften at least one component of the web is directed through the web; chemical bonding using, for example, latex adhesives that are deposited onto the web by, for example, spraying; and consolidation by mechanical methods such as needling and hydroentanglement. "Carded thermobonded" nonwoven thus refers to a carded nonwoven wherein the bonding is achieved by use of heat. "Carded thermobonded by calendering nonwoven" thus refers to a carded nonwoven wherein the bonding is achieved by use of heat in a calendering process, wherein a web of loose fibers is thermally bonded by passing the fibers through the nip of a pair of calender rollers, of which one or both are heated (plain or patterned roller may be used).

As used herein, the term "cellulosic" is meant to include any material having cellulose as a major constituent, and specifically comprising at least 50 percent by weight cellulose or a cellulose derivative. Thus, the term includes cotton, typical wood pulps, nonwoody cellulosic fibers, cellulose acetate, cellulose triacetate, rayon, thermomechanical wood pulp, chemical wood pulp, debonded chemical wood pulp, milkweed, or bacterial cellulose.

"Coform" as used herein is intended to describe a blend of meltblown fibers and cellulose fibers that is formed by air forming a meltblown polymer material while simultaneously blowing air-suspended cellulose fibers into the stream of meltblown fibers. The coform material may also include other materials, such as superabsorbent particles. The meltblown fibers containing wood fibers are collected on a forming surface, such as provided by a foraminous belt. The forming surface may include a gas-pervious material, such as spunbonded fabric material, that has been placed onto the forming surface.

"Compression" refers to the process or result of pressing by applying force on an object, thereby increasing the density of the object.

The term "consisting essentially of" does not exclude the presence of additional materials which do not significantly affect the desired characteristics of a given composition or product. Exemplary materials of this sort would include, without limitation, pigments, antioxidants, stabilizers, surfactants, waxes, flow promoters, solvents, particulates and materials added to enhance processability of the composition.

"Conventional hot-melt adhesive" means a formulation that generally comprises several components. These components typically include one or more polymers to provide cohesive strength (e.g., aliphatic polyolefins such as poly (ethylene-co-propylene) copolymer; ethylene vinyl acetate copolymers; styrene-butadiene or styrene- isoprene block copolymers; etc.); a resin or analogous material (sometimes called a tackifier) to provide adhesive strength (e.g., hydrocarbons distilled from petroleum distillates; rosins and/or rosin esters; terpenes derived, for example, from wood or citrus, etc.); perhaps waxes, plasticizers or other materials to modify viscosity (i.e., flowability) (examples of such materials include, but are not limited to, mineral oil, polybutene, paraffin oils, ester oils, and the like); and/or other additives including, but not limited to, antioxidants or other stabilizers. A typical hot-melt adhesive formulation might contain from about 15 to about 35 weight percent cohesive strength polymer or polymers; from about 50 to about 65 weight percent resin or other tackifier or tackifiers; from more than zero to about 30 weight percent plasticizer or other viscosity modifier; and optionally less than about 1 weight percent stabilizer or other additive. It should be understood that other adhesive formulations comprising different weight percentages of these components are possible.

The term "density" or "concentration" when referring to the absorbent material, in particular the SAP, of a layer, refers to the amount of the absorbent material divided by the surface area of the layer over which the absorbent material is spread out.

The term "disposable" is used herein to describe absorbent articles that generally are not intended to be laundered or otherwise restored or reused as an absorbent article (i.e., they are intended to be discarded after a single use and, preferably, to be recycled, composted or otherwise disposed of in an environmentally compatible manner).

As used herein, the terms "elastic", "elastomeric", "elasticity" or derivations thereof are used to describe the ability of various materials and objects comprised of such to reversibly undergo deformation under stress, e.g., become stretched or extended, in at least one direction when a force is applied to the material and to resume substantially to their original dimensions upon relaxing, i.e., when the force is released, without rupture or breakage. Preferably, it refers to a material or composite which can be elongated in at least one direction by at least 50% of its relaxed length, i.e., elongated to at least 150% of its relaxed length, and which will recover upon release of the applied tension at least 40% of its elongation. Accordingly, upon release of the applied tension at 50% elongation, the material or composite contracts to a relaxed length of not more than 130% of its original length. Examples of suitable elastomer materials include polyether-polyamide block copolymers, polyurethanes, synthetic linear A-B-A and A-B block copolymers, chlorinated rubber/EVA (ethylene-vinyl acetate) blends, EPDM (ethylene-propylene diene monomer) rubbers, EPM (ethylene-propylene monomer) rubbers, blends of EPDM/EPM/EVA, and the like.

The term "elasticized" refers to a material, layer, or substrate that is naturally non-elastic, but which has been rendered elastic by, for example, suitably joining an elastic material, layer, or substrate thereto.

"Elongation" means the ratio of the extension of a material to the length of the material prior to the extension (expressed in percent), as represented by the following: "Extension" means the change in length of a material due to stretching (expressed in units of length).

As used herein the term "extensible" means elongatable in at least one direction, but not necessarily recoverable.

The term "fabrics" is used to refer to all of the woven, knitted and nonwoven fibrous webs.

As used herein, the term "garment" means any type of apparel which may be worn. This includes diapers, training pants, incontinence products, surgical gowns, industrial workwear and coveralls, undergarments, pants, shirts, jackets and the like.

Many of the known superabsorbent polymer particles exhibit gel blocking. "Gel blocking" occurs when superabsorbent polymer particles are wetted and the particles swell so as to inhibit fluid transmission to other regions of the absorbent structure. Wetting of these other regions of the absorbent member therefore takes place via a very slow diffusion process. In practical terms, this means acquisition of fluids by the absorbent structure is much slower than the rate at which fluids are discharged, especially in gush situations. Leakage from the absorbent article can take place well before the particles of SAP in the absorbent member are even close to being fully saturated or before the fluid can diffuse or wick past the "blocking" particles into the rest of the absorbent member. Gel blocking can be a particularly acute problem if the superabsorbent polymer particles do not have adequate gel strength and deform or spread under stress once the particles swell with absorbed fluid.

The term "graphic" includes, but is not limited to, any type of design, image, mark, figure, codes, words, patterns, or the like. For a product such as a training pant, graphics will generally include objects associated with little boys and little girls, such as multi-color trucks, airplanes, balls, dolls, bows, or the like.

"Hydroentanglement process" refers to the manufacturing of nonwoven webs. The process involves directing a series of water jets towards a fibrous web which is supported on a moving porous belt. The water jets pass downwards through the mass of fibres and on making contact with the surface of the belt, the jets rebound, and break up: the energy released causes entanglement of the mass of fibres.

The term "hydrophilic" describes fibers or the surfaces of fibers which are wetted by the aqueous liquids in contact with the fibers. The degree of wetting of the materials can, in turn, be described in terms of the contact angles and the surface tensions of the liquids and materials involved. The term "wettable" is meant to refer to a fiber which exhibits a liquid, such as water, synthetic urine, or a 0.9 weight percent aqueous saline solution, in air contact angle of less than 90°, whereas "hydrophobic" or "non-wettable" describes fibers having contact angles equal to or greater than 90°.

As used herein, the term "impermeable" generally refers to articles and/or elements that are substantially not penetrated by aqueous fluid through the entire thickness thereof under a pressure of 1.0 kPa or less. Preferably, the impermeable article or element is not penetrated by aqueous fluid under pressures of 3.4 kPa or less. More preferably, the impermeable article or element is not penetrated by fluid under pressures of 6.8 kPa or less. An article or element that is not impermeable is permeable.

"Join", "joining", "joined", or variations thereof, when used in describing the relationship between two or more elements, means that the elements can be connected together in any suitable manner, such as by heat sealing, ultrasonic bonding, thermal bonding, by adhesives, stitching, or the like. Further, the elements can be joined directly together, or may have one or more elements interposed between them, all of which are connected together.

"Laminate" refers to elements being attached together in a layered arrangement.

The use of the term "layer" can refer, but is not limited, to any type of substrate, such as a woven web, nonwoven web, films, laminates, composites, elastomeric materials, or the like. A layer can be liquid and air permeable, permeable to air but impermeable to liquids, impermeable both to air and liquid, or the like. When used in the singular, it can have the dual meaning of a single element or a plurality of elements.

The crotch portion of the absorbent article preferably comprises opposite longitudinal side portions which comprise a pair of elasticized, longitudinally-extending "leg cuffs". The leg cuffs are generally adapted to fit about the legs of a wearer when in use and serve as a mechanical barrier to the lateral flow of body exudates. Leg cuffs are elasticized by leg elastics. The diaper further can comprise a front waist elastic and a rear waist elastic. Materials suitable for use in forming leg elastics are known to those skilled in the art. Exemplary of such materials are strands or ribbons of a polymeric, elastomeric material which are adhered to the diaper at the leg cuff while in a stretched position, or which are attached to the diaper while the diaper is pleated, such that elastic constrictive forces are imparted to the leg cuff. Examples of suitable elastomer materials that can be used include polyether-polyamide block copolymers, polyurethanes, synthetic linear A-B-A and A-B block copolymers, chlorinated rubber/EVA (ethylene-vinyl acetate) blends, EPDM (ethylene-propylene diene monomer) rubbers, EPM (ethylene-propylene monomer) rubbers, blends of EPDM/EPM/EVA, and the like.

"Liquid" means a nongaseous substance and/or material that flows and can assume the interior shape of a container into which it is poured or placed.

"Longitudinal" is a direction running parallel to the maximum linear dimension of the article.

The term "meltblown fibers" means fibers formed by extruding a molten thermoplastic material through a plurality of fine, usually circular, die capillaries as molten threads or filaments into a high velocity gas stream (e.g. air) which attenuates the filaments of molten thermoplastic material to reduce their diameter, which may be to microfiber diameter. In general, meltblown fibers have an average fiber diameter of up to about 10 microns. After the fibers are formed, the meltblown fibers are carried by the high velocity gas stream and are deposited on a collecting surface to form a web of randomly disbursed meltblown fibers.

The term "nonelastic" refers to any material which does not fall within the definition of "elastic" above The term "nonwoven fabric or web" means a sheet material having a structure of individual fibers or threads which are interlaid, but not in a regular manner such as occurs with knitting or weaving processes. Nonwoven fabrics or webs have been formed from many processes such as for example, meltblowing processes, spunbonding processes, and bonded carded web processes.

"Pant body" or "pant" refers to a garment that has a waist opening and a pair of leg openings, similar to shorts, swim wear, or the like. The described garment may or may not have a manually tearable side seam.

By the terms "particle", "particles", "particulate", "particulates" and the like, it is meant that the material is generally in the form of discrete units. The units can comprise granules, powders, spheres, pulverized materials or the like, as well as combinations thereof. The particles can have any desired shape such as, for example, cubic, rod-like, polyhedral, spherical or semi-spherical, rounded or semi-rounded, angular, irregular, etc. Shapes having a large greatest dimension/smallest dimension ratio, like needles, flakes and fibers, are also contemplated for inclusion herein. The terms "particle" or "particulate" may also include an agglomeration comprising more than one individual particle, particulate or the like. Additionally, a particle, particulate or any desired agglomeration thereof may be composed of more than one type of material.

The term "polymer" generally includes, but is not limited to, homopolymers, copolymers, such as, for example, block, graft, random and alternating copolymers, terpolymers, etc. and blends and modifications thereof. Furthermore, unless otherwise specifically limited, the term "polymer" shall include all possible geometrical configurations of the material. These configurations include, but are not limited to, isotactic, syndiotactic and random symmetries.

"Pulp fluff" or "fluff pulp" refers to a material made up of cellulose fibers. The fibers can be either natural or synthetic, or a combination thereof. The material is typically lightweight and has absorbent properties.

The "retention portion" or "liquid absorption layer" is part of the absorbent medium. This portion may comprise a matrix of hydrophilic fibers, such as a web of cellulosic fluff, mixed with particles of high absorbency material. In particular arrangements, the retention portion may comprise a mixture of superabsorbent hydrogel-forming particles and synthetic polymer meltblown fibers, or a mixture of superabsorbent particles with a fibrous coform material comprising a blend of natural fibers and/or synthetic polymer fibers. The superabsorbent particles may be substantially homogeneously mixed with the hydrophilic fibers, or may be nonuniformly mixed. For example, the concentrations of superabsorbent particles may be arranged in a non-step-wise gradient through a substantial portion of the thickness of the absorbent structure, with lower concentrations toward the bodyside of the absorbent structure and relatively higher concentrations toward the outside of the absorbent structure. The superabsorbent particles may also be arranged in a generally discrete layer within the matrix of hydrophilic fibers. In addition, two or more different types of superabsorbent may be selectively positioned at different locations within or along the fiber matrix.

As used herein the term "sheet" or "sheet material" refers to woven materials, nonwoven webs, polymeric films, polymeric scrim-like materials, and polymeric foam sheeting.

The term "spunbond fibers (or layer(s) or nonwovens)" refers to fibers formed by extruding molten thermoplastic polymers as filaments or fibers from a plurality of relatively fine, usually circular, capillaries of a spinneret, and then rapidly drawing the extruded filaments by an eductive or other well-known drawing mechanism to impart molecular orientation and physical strength to the filaments. The average diameter of spunbond fibers is typically in the range of from 15-60 $\mu$m or higher. The spinneret can either be a large spinneret having several thousand holes per meter of width or be banks of smaller spinnerets, for example, containing as few as 40 holes.

The term "spunbond meltblown spunbond" (SMS) nonwoven fabric as used herein refers to a multi-layer composite sheet comprising a web of meltblown fibers sandwiched between and bonded to two spunbond layers. A SMS nonwoven fabric can be formed in-line by sequentially depositing a first layer of spunbond fibers, a layer of meltblown fibers, and a second layer of spunbond fibers on a moving porous collecting surface. The assembled layers can be bonded by passing them through a nip formed between two rolls that can be heated or unheated and smooth or patterned. Alternately, the individual spunbond and meltblown layers can be pre-formed and optionally bonded and collected individually such as by winding the fabrics on wind-up rolls. The individual layers can be assembled by layering at a later time and bonded together to form a SMS nonwoven fabric. Additional spunbond and/or meltblown layers can be incorporated to form laminate layers, for example spunbond-meltblown-meltblown-spunbond (SMMS), or spunbond-meltblown (SM) etc.

"Staple fibers" refer to commercially available fibers having diameters ranging from less than about 0.001 mm to more than about 0.2 mm; they come in several different forms such as short fibers ranging from about 10 to 50 mm in length and long fibers with a length higher than 50 mm, preferably up to 100 mm.

By "stretch", it is meant that the material has the ability to extend beyond its original size in at least one dimension when subjected to a tensile force (i. e., tension) applied in the direction of that dimension, without breaking the material. An extension of for example 50% means that the material with an initial length of 100mm has reached a length of 150mm. Stretch may be unidirectional, bi-directional, or multi-directional. The specific stretch properties of a material may vary along any of the stretch vectors. The term can include elastic materials, as well as nonwovens that can be inherently extensible, but not necessarily in an elastic manner. Such nonwovens can be made to behave in an elastic manner by bonding them to elastic films.

As used herein "channels" are fluid distribution means within the absorbent core adapted to favour exudate flow there along and are typically intended to exclude embossing patterns or ducts formed by compression from the meaning thereof and rather include structures that are substantially free of absorbent material instead of comprising compacted absorbent material. Channels herein are formed by joining upper and lower layers of a core wrap as will be described in more detail hereinbelow. Channels preferably comprise recessed regions forming visible conduits or passages typically extending along the longitudinal axis of the core and having a depth in a direction perpendicular to said longitudinal axis. By "visible" it is herein intended clearly visible by naked eye. Typically the channels have a width generally greater than 1mm, preferably from 5mm to 50mm, more preferably from 6mm to 40mm, more preferably from 8mm to 30mm, even more preferably from greater than 8mm to less than 25mm.

By "substantially", it is meant at least the majority of the structure referred to. For example, with reference to a channel following "a substantially continuous path from any point of said channel to any other point of the same channel", this means that the majority of the channel is interconnected and generally wherein a direct and continuous path can be traced by starting from one point of the channel towards another point of the channel.

By "directly over", it is meant that the feature referred to is placed over the structure referred to such that the two are in direct contact with each other at least throughout a substantial portion of said structure.

By "indirectly over", it is meant that the feature referred to is placed over the structure referred to but in such a way that the two are not in direct contact with each other at least throughout a substantial portion of said structure. For example, a nonwoven web applied indirectly over a three-dimensional absorbent material comprises a further layer of material between said nonwoven web and said three-dimensional absorbent material.

Use of the term "substrate" includes, but is not limited to, woven or nonwoven webs, porous films, ink permeable films, paper, composite structures, or the like.

The terms "Superabsorbent" or "high absorbency" refer to materials that are capable of absorbing at least 10 times their own weight in liquid. Superabsorbent materials suitable for use in the present disclosure are known to those skilled in the art, and may be in any operative form, such as particulate form, fibers and mixtures thereof. Generally stated, the "superabsorbent material" can be a water-swellable, organic or inorganic, generally water-insoluble, hydrogel-forming polymeric absorbent material, which is capable of absorbing at least about 15, suitably about 30, and possibly about 60 times or more its weight in physiological saline (e.g. saline with 0.9 wt % NaCl). The superabsorbent material may be biodegradable or bipolar. The hydrogel-forming polymeric absorbent material may be formed from organic hydrogel-forming polymeric material, which may include natural material such as agar, pectin, and guar gum; modified natural materials such as carboxymethyl cellulose, carboxyethyl cellulose, and hydroxypropyl cellulose; and synthetic hydrogel-forming polymers. Synthetic hydrogel-forming polymers include, for example, alkali metal and ammonium salts of polyacrylic acid and polymethacrylic acid, polyacrylamides, polyvinyl alcohol, ethylene maleic anhydride copolymers, polyvinyl ethers, polyvinyl morpholinone, polymers and copolymers of vinyl sulfonic acid, polyacrylates, polyacrylamides, polyvinyl pyridine, maleic anhydride copolymers with vinyl ethers and alpha-olefins, poly(vinyl pyrrolidone), poly(vinylmorpholinone), poly(vinyl alcohol), and the like, and mixtures and copolymers thereof. Other suitable hydrogel-forming polymers include hydrolyzed acrylonitrile grafted starch, acrylic acid grafted starch, and isobutylene maleic anhydride copolymers and mixtures thereof, methyl cellulose, carboxymethyl cellulose, hydroxypropyl cellulose, and the natural gums, such as alginates, xanthan gum, locust bean gum and the like. The hydrogel-forming polymers may be inorganic materials, such as silica gels, or organic compounds such as cross-linked polymers. The term "cross-linked" refers to any means for effectively rendering normally water-soluble materials substantially water insoluble but swellable. Such means can include, for example, physical entanglement, irradiation, crystalline domains, covalent bonds, ionic complexes and associations, hydrophilic associations, such as hydrogen bonding, and hydrophobic associations or Van der Waals forces. Mixtures of natural and wholly or partially synthetic absorbent polymers can also be used. Synthetic hydrogel-forming materials typically are xerogels which form hydrogels when wetted. The term "hydrogel", however, has commonly been used to also refer to both the wetted and unwetted forms of the material. The superabsorbent material may be in any of a wide variety of geometric forms. As a general rule, it is preferred that the superabsorbent material be in the form of discrete particles. However, the superabsorbent material may also be in the form of fibres, flakes, rods, spheres, needles, spiral or semi-spiral, cubic, rod-like, polyhedral, or the like. Conglomerates of particles of superabsorbent material may also be used. The superabsorbent material may suitably be included in an appointed storage or retention portion of the absorbent system, and may optionally be employed in other components or portions of the absorbent article. The superabsorbent material may be included in the absorbent layer or other fluid storage layer of the absorbent article in an amount of from about 5 to about 100 weight percent and desirably from about 30 to about 100 weight percent based on the total weight of the absorbent core. The distribution of the high-absorbency material within the different portions of the absorbent core can vary depending upon the intended end use of the absorbent core. The high-absorbency material may be arranged in a generally discrete layer within the matrix of hydrophilic fibres. Alternatively, the absorbent core may comprise a laminate of fibrous webs and high-absorbency material or other suitable means of maintaining a high-absorbency material in a localized area.

"Superabsorbent polymer particles" or "SAPs" refer to water-swellable, water-insoluble organic or inorganic materials capable, under the most favorable conditions, of absorbing at least about 10 times their weight, or at least about 15 times their weight, or at least about 25 times their weight in an aqueous solution containing 0.9 weight percent sodium chloride. In absorbent articles, such as diapers, incontinent diapers, etc., the particle size is typically ranging between 100 to 800 $\mu$m, preferably between 300 to 600 $\mu$m, more preferably between 400 to 500 $\mu$m.

"Tension" includes a uniaxial force tending to cause the extension of a body or the balancing force within that body resisting the extension.

As used herein, the term "thermoplastic" is meant to describe a material that softens when exposed to heat and which substantially returns to its original condition when cooled to room temperature.

The term "top sheet" or "topsheet" refers to a liquid permeable material sheet forming the inner cover of the absorbent article and which in use is placed in direct contact with the skin of the wearer. The top sheet is typically employed to help isolate the wearer's skin from liquids held in the absorbent structure. The top sheet can comprise a nonwoven material, e.g. spunbond, meltblown, carded, hydroentangled, wetlaid etc. Suitable nonwoven materials can be composed of man-made fibres, such as polyester, polyethylene, polypropylene, viscose, rayon etc. or natural fibers, such as wood pulp or cotton fibres, or from a mixture of natural and man-made fibres. The top sheet material may further be composed of two fibres, which may be bonded to each other in a bonding pattern. Further examples of top sheet materials are porous foams, apertured plastic films, laminates of nonwoven materials and apertured plastic films etc. The materials suited as top sheet materials should be soft and non-irritating to the skin and be readily penetrated by body fluid, e.g. urine or menstrual fluid. The inner coversheet may further be different in different parts of the absorbent article. The top sheet fabrics may be composed of a substantially hydrophobic material, and the hydrophobic material may optionally be treated with a surfactant or otherwise processed to impart a desired level of wettability and hydrophilicity.

"Training pants" are available for use by children in the potty-training stage, and are popular with mothers and caretakers. A training pant typically comprises a top sheet, a back sheet, an absorbent medium between the top sheet and the back sheet, and side seams that bond portions of the side edges of the pant together to form waist and leg openings.

As used herein, the term "transverse" or "lateral" refers to a line, axis, or direction which lies within the plane of the absorbent article and is generally perpendicular to the longitudinal direction.

"Ultrasonic welding or bonding" refers to a technology which joins two materials by melting them with heat generated from ultrasonic oscillation and then laminating them together, such that the molten materials flow and fill the gap between the two unaffected portions of the two materials, respectively. Upon cooling and shaping, the two materials are joined together.

"Dry-state" refers to the condition in which an absorbent article has not yet been saturated with exudates and/or liquid.

"Wet-state" refers to the condition in which an absorbent article has been saturated with exudates and/or liquid. Typically wherein at least 30ml, preferably at least 40ml, even more preferably at least 50ml, most preferably from 60ml to 800ml, of exudate and/or liquid are contained in the absorbent article.

As used herein, the term "water-swellable, water-insoluble" is meant to refer to a material that, when exposed to an excess of water, swells to its equilibrium volume but does not dissolve into the solution. As such, a water-swellable, water-insoluble material generally retains its original identity or physical structure, but in a highly expanded state, during the absorption of the water and, thus, must have sufficient physical integrity to resist flow and fusion with neighboring particles.

By the term "concentration" of (e.g. super absorbent polymer) as used herein, is meant the amount of the referred material divided by the surface area of the layer referred to (typically said area being in the plane of the length and width of the core) over or within which the referred material is contained. This may be determined by standard weighing and dimensional measuring methods known in the art and can be expressed in g/mm$^2$.

By the term "substantially U-shaped" as used herein, is meant any shape that visually approximates the shape of a "U", such as a "V-shape", a semi-circle, and the like.

By the term "distinct cellulosic fibers" as used herein, is meant cellulosic fibers that are not part of a substrate (e.g. a nonwoven layer) and are rather distinct thereof and/or physically separated therefrom, and typically are in the form of cellulosic fibers that are enclosed though kept separate from said substrate. For sake of clarity, cellulosic fibers present within a substrate (e.g. a nonwoven layer) are not encompassed within its meaning.

By the term "substantially matches the shape of the channel(s)" as used herein, is meant that the feature referred to has an overlapping shape that is visually the same as the channel(s).

The term "coincident" as used herein has its standard meaning as provided in dictionaries and means having the same position in space or occupying the same space. When talking about two surfaces, two objects or two shapes, coincident means corresponding point to point, being able to overlap, to cover each other.

By "superposed", it is meant that the two elements are positioned vertically congruent or coincident in a vertical relation when the article is considered in a flattened configuration. The superposed components may be in direct or indirect contact.

By the term "superabsorbent polymer fibers" as used herein, is meant fibers made from superabsorbent polymers (as opposed to particles made therefrom). Examples of suitable fibers for use herein are selected from those of Example 1, Example 2, Example 3, and/or Example 4 (page 5, lines 1-46) of EP3190216A1, incorporated herein by reference. Said fibers typically being used to form nonwoven webs or substrates according to the same referenced application.

By the terms "longitudinally-, transversally-, diagonally- -extending" as used herein, is meant a general orientation substantially parallel respectively to the longitudinal axis, to the transversal axis, and to any axis between them. This covers deviations from the axis used as reference of between 0° and 20°. This may cover straight lines but also curved lines. In the latter case, it is the main general orientation of the curve which is meant to be described.

By the term "center line" as used herein, is meant an axis dividing the element to which it refers, in two portions of equal length on either side of this axis.

Embodiments of the articles and processes according to the disclosure will now be described. It is understood that technical features described in one or more embodiments maybe combined with one or more other embodiments without departing from the intention of the disclosure and without generalization therefrom.

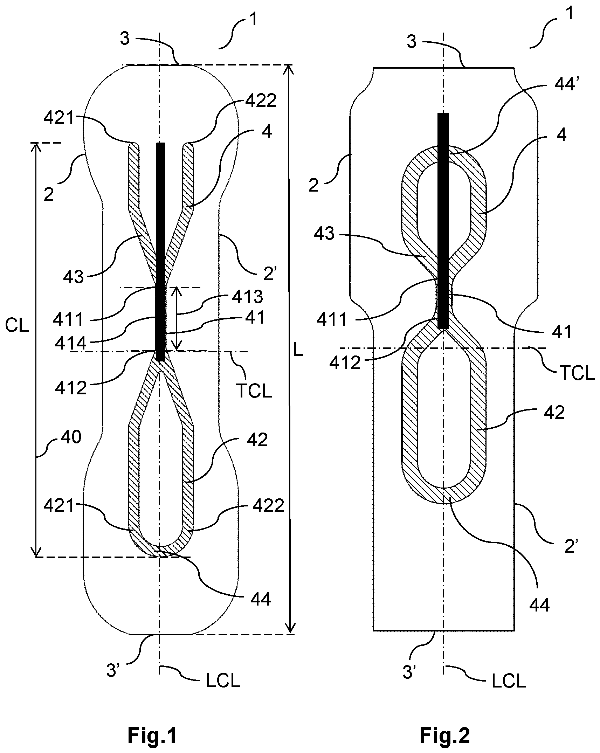

Absorbent articles according to the invention comprise a liquid permeable topsheet, a liquid impermeable backsheet and an absorbent core positioned in between said topsheet and said backsheet. The absorbent core (1), as for example illustrated in Figure 1, has a first and second longitudinal edge (2,2') and a front and back transverse edge (3,3'). It has a longitudinal center line (LCL) dividing the absorbent core (1) in a first longitudinal portion and a second longitudinal portion on either side of the longitudinal center line (LCL). It also has a transverse center line (TCL) dividing the absorbent core (1) in a front portion and a back portion on either side of the transverse center line (TCL). The longitudinal center line (LCL) and the transverse center line (TCL) together create four quadrants within the absorbent core (1). The absorbent core (1) comprises absorbent material selected from the group consisting of cellulose fibers, superabsorbent polymers and combinations thereof, and the absorbent material is contained within at least one core wrap substrate enclosing said absorbent material. The top layer of said core wrap is adhered to a bottom layer of said core wrap to form one or more channels (4) substantially free of said absorbent material. At least one channel (4) follows a substantially continuous path from any point of said channel to any other point of the same channel and comprises at least one longitudinally extending channel central section (41) coincident with the longitudinal center line (LCL) and closest to or crossing the transverse center line (TCL). The absorbent articles further comprise a continuously extending wetness indicator (5), which as seen from the garment-facing side of the article, is coincident with the longitudinal center line (LCL). Although the wetness indicator may not be part of the absorbent core itself, the location of the wetness indicator is drawn in Figures 1-12 either as a black rectangle when the channel is hatched or as a hatched rectangle when the channel is black. The wetness indicator (5) comprises an overlapping portion (51) superposed with at least part of the longitudinally extending channel central section (41), and an exposed portion (52) superposed with absorbent material at least partially surrounded by channel sections (42,43) closer to the front transverse edge (3).

The wetness indicator changes appearance when contacted with liquid bodily exudates, for example urine, and may comprise an appearing signal, a disappearing signal or a colour change signal that changes appearance when contacted with liquid to allow a caregiver to verify whether the article is wet.

The wetness indicator may typically be placed towards the bottom side of the core, in particular between the bottom side of the core and the internal surface of the liquid impermeable backsheet. By "as seen from the garment-facing side of the article" it is meant that although the wetness indicator (5) may or may not be placed directly in the plane of the channel (4) or even within the absorbent core (1), when considering the absorbent article from the outside, that is typically looking at the outward surface of the backsheet, the wetness indicator seems partially superposed with the channel and partially superposed with absorbent material. As the channel is typically visible from the outside of the article when the article is sufficiently loaded with urine, the wetness indicator also typically appears placed partially within the channel and partially out of the channel in the loaded article when seen from the exterior of the article. The word "seems" should be construed herein in a broad sense, as in some embodiments, the wetness indicator may comprise or consists of an appearing signal or a disappearing signal, so that the wetness indicator is only visible to an observer in the wet or dry state, as will be exemplified further below.

The wetness indicators of the present invention may be according to any wetness indicating system known in the art. It is known that wetness indicator can provide an appearing signal, a disappearing signal or a colour change signal, and of course combinations thereof. An appearing signal will typically not be visible or more generally perceivable in the dry article, and becomes visible or otherwise perceivable when the article is wet. An appearing signal may for example be provided by a composition which is transparent or having a colour that matches the colour of the backsheet material in its dry state, and then changes to a different colour when contacted with urine. The wetness indicator of the invention may also provide a disappearing signal when the article is wet. A disappearing signal may be provided by a composition that has a first colour when dry and which changes to a second colour that matches the general colour of the backsheet or any graphic printed on the backsheet, so that the second colour is less discernible that the first colour on the article. Such a disappearing signal may be provided for example by a composition comprising a dye that dissolves in urine and thus fades as the article is wetted. The wetness indicator may advantageously provide a colour change signal, which may be typically obtained by a composition having a first colour when dry and a second colour different form the first colour when wet, both colours being discernible by an external observer considering the article in a dry and a wet state. The wetness indicator may in particular be a colour change composition comprising a suitable pH indicator or another chemical substance that changes colour when contacted with urine.

The wetness indicator may be a hot-melt adhesive, which allows for an easy application of the composition on a substrate component of the article for example by a slot coating process or printed adhesive coating. The wetness indicator may be an ink, which allows for an easy application of the composition on a substrate component of the article for example by a printing process.

The wetness indicator composition may be applied on any layer of the absorbent article using a conventional technique, for example printing, spraying or coating, during the making of the absorbent article. The layer may advantageously be the inner surface of the backsheet or the outer surface of the bottom side of the core wrap. This allows the wetness indicator to be visible from the exterior of the article by transparency through the backsheet while keeping the wetness indicator composition within the article. The wetness indicator may in particular be easily applied on a layer such a nonwoven or film by a slot-coating process especially if the composition can be applied as a hot-melt. The slot-coating process allows applying a well-defined slot or a series of slots extending in the machine direction of the converting line, which is typically parallel to the longitudinal direction of the article.

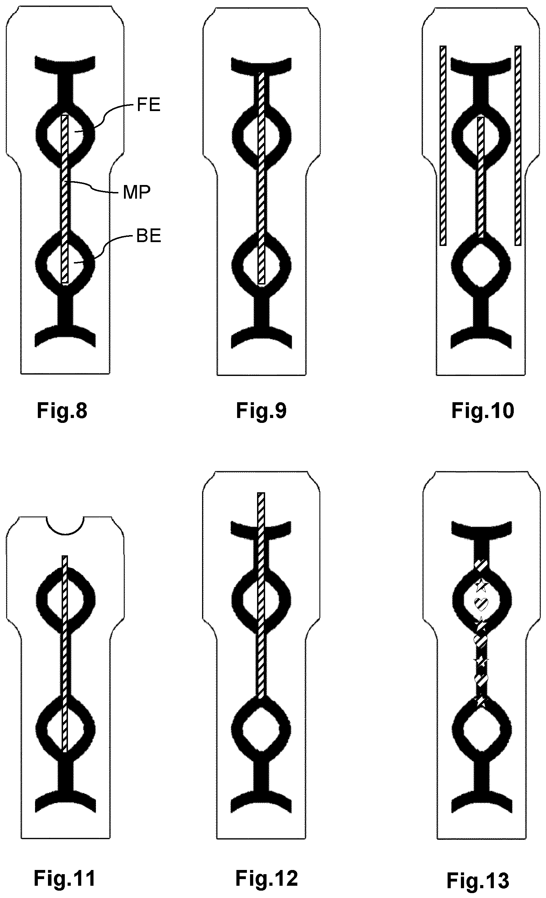

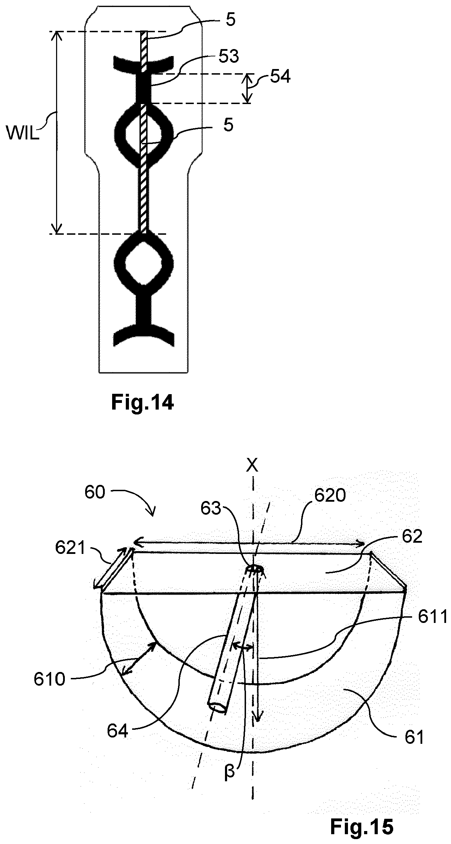

By "continuously extending wetness indicator coincident with the longitudinal center line (LCL)" we mean herein that the wetness indicator has a generally elongated shape substantially parallel and superposed with the longitudinal center line (LCL), but it may comprise individual and discrete elements which together create this generally elongated shape, e.g. letters and numbers creating a code or a word; decorative elements like animal shapes; geometrical shapes such as triangles, stars, squares or circles; or object shapes like toys, cars, etc., as illustrated in Figure 13. The continuity of the generally elongated shape of the wetness indicator may be interrupted by one or more interruption zones (53) without wetness indicator material or composition, but such zones are then limited to a length (54) parallel to the longitudinal center line (LCL) of less than 40% or 30%, preferably less than 25%, more preferably less than 20%, most preferably less than 15% of the total length of the wetness indicator (WIL), as illustrated in Figure 14. The total length (WIL) of the wetness indicator corresponds to the maximum extension of the wetness indicator along the longitudinal center line (LCL), optionally including one or more zones (53) without wetness indicator material or composition. The total length (WIL) of the wetness indicator can be between 30 and 70%, more preferably between 40 and 60%, most preferably between 45 and 55%, of the absorbent core length (L).

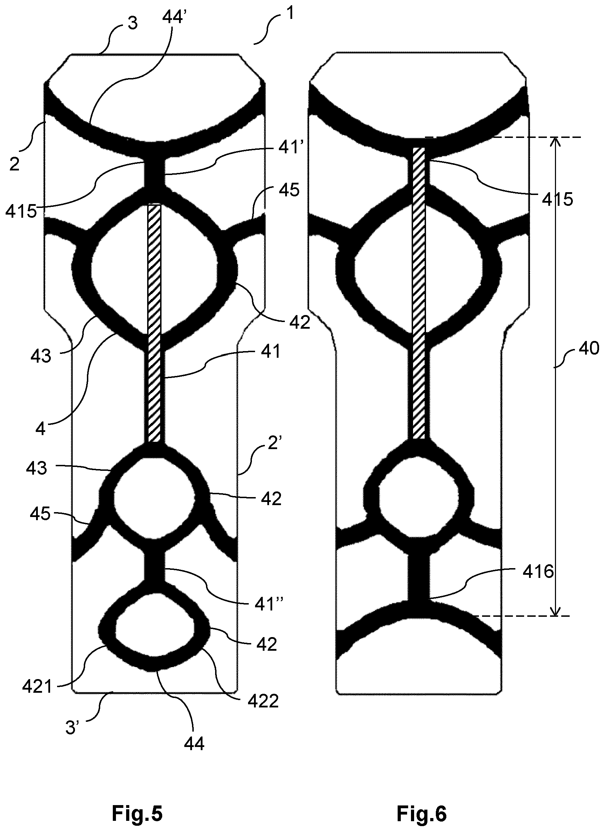

As visible on the various examples of wetness indicator placements illustrated in Figures 1 to 14, the wetness indicator positioning is at least partially overlapping the longitudinally extending channel central section (41) and at least partially overlapping absorbent material closer to the front transverse edge (3). Said material closer to the front transverse edge being superposed by the wetness indicator exposed portion (52) is at least partially surrounded by channel sections, in particular by the longitudinally extending lateral sections (42) and the diagonally extending diagonal sections (43) of the channel and optionally the transversally extending ending section (44). The wetness indicator may be superimposed with the channel until the channel front extremity like in Figures 1 or 6. It may go beyond the channel front extremity and protrude towards the front transverse edge (3) like in Figures 12 and 14. Preferably a distance is kept free of wetness indicator in the front part of the article close to the front transverse edge (3) and the wetness indicator does not extend to the front transverse edge (3). This zone free of wetness indicator in the front part of the article may correspond to the positioning of the frontal tape for example used in diapers as a landing zone for the closing of the back ears of the diaper on the belly part of the diaper. An overlap of the wetness indicator and the frontal tape could be interpreted as a manufacturing misplacement of the wetness indicator and/or could be useless if the frontal tape does not allow to see through the wetness indicator. The same preference applies to absorbent core intended for newborn diapers with umbilical cord notch-cutout, as illustrated in Figure 11.

In instances where the front ending section (44') of the channel is an arc-shaped closing section with the convex side of the arc facing the front transverse edge (3), it might be preferred that the wetness indicator extends beyond the channel front extremity, as illustrated for example in Figures 2, to ensure that the absorbent article is not changed too early and that the absorbent material in front of the channel get wetted and the corresponding wetness indicator portion changes appearance before the change. This might be preferred for articles of larger sizes, when the distance between the front extremity of the channel and the front transverse edge, is significant enough.

The width of the wetness indicator may be similar to the width of the channel, or narrower, as long as clearly visible by the user, or wider, but preferably not using too much wetness indicator composition to limit costs. Preferably the width of the wetness indicator is between 2 and 8 mm, more preferably between 3 and 6 mm.

The absorbent article may comprise one or more additional wetness indicator(s). These may give additional information about the fullness of the absorbent article. Figure 10, for example, shows two additional longitudinal wetness indicators, parallel to the longitudinal center line (LCL) on both sides of the channel, outside of the channel. These may help in giving additional information regarding fullness of the article close to the longitudinal edges, thereby allowing the article to be changed in time and limiting risk of leakage through the sides of the article.

Advantageously, the opacity of the layers above (i.e. closer to the wearer-facing side of the article than) the wetness indicator overlapping portion (51), such as top and/or bottom core wrap(s), optional ADL, adhesive and topsheet, is such as to allow to see the wetness indicator overlapping portion (51) through said layers, viewed from the wearer-facing side of the article. This may facilitate the monitoring and quality control of the manufacturing process, allowing to visually control, for example with cameras, the proper placement of the wetness indicator.

The at least one channel (4) may comprise at least:

- one longitudinally extending central section (41) coincident with the longitudinal center line (LCL) closest to or crossing the transverse center line (TCL),
- one longitudinally extending lateral section (42) in each of the four quadrants,
- four diagonally extending diagonal sections (43), diverging from the central section end points (411,412) towards the lateral sections in each quadrant and connecting said central (41) and lateral sections (42), and
- one transversally extending ending section (44,44') in one of the front or back portions, closest to the absorbent core respective front or back transverse edge (3,3').

This may allow exceptionally fast absorption and good liquid distribution throughout the entire core, leading to improved absorption capacity and low rewet that provides to the consumer an enhanced perception of dryness. It further may offer an improved fit to the wearer, thereby providing comfort and minimizing the risk of leakage.

In some embodiment, as for example illustrated in Figures 2 to 7, the channel (4) comprises a second transversally extending ending section (44,44'), in the other of the front or back portions, closest to the absorbent core respective front or back transverse edge (3,3'). This may allow distribution of fluid beyond the second ending section itself, closer to the transverse edge of the core.

The transversally extending ending section (44,44') may be a closing section joining each of two lateral sections end points (421,422) closest to the absorbent core respective front or back transverse edge (3,3'), as for example illustrated in Figures 1 to 4. In said case, preferably, the ending section is arc-shaped with the convex side of the arc facing the front or back transverse edge (3,3'). Alternatively, the transversally extending ending section (44,44') according to the invention may be connected centrally to an end point (415,416) of a longitudinally extending central section (41) closest to the absorbent core respective front or back transverse edge (3,3'), as for example illustrated in Figures 5 to 7. In said alternative, preferably, the ending section (44,44') is arc-shaped with the concave side of the arc facing the front or back transverse edge (3,3') and/or the ending section (44,44') extends until the longitudinal edges (2,2') of the absorbent core (1).

In an embodiment, as illustrated in Figure 1, the channel (4) consists of one central section (41), four lateral sections (42), four diagonal sections (43) and one closing section (44). In that case, preferably, the closing section is in the back portion of the absorbent core, since this portion is generally the most difficult to distribute liquid as being the farthest from the miction point.

In another embodiment, as illustrated in Figure 2, the channel (4) consists of one central section (41), four lateral sections (42), four diagonal sections (43) and two closing sections (44,44'), in order to benefit from the advantages of the closing sections hereinabove described, in both the front and the back of the absorbent core.

In these embodiments where the channel consists of one central section, four lateral sections, four diagonal sections and one or two closing section(s), the central section is preferably longitudinally centered on a point (414) which is at a distance from the front transverse edge (3) along the longitudinal center line (LCL) of between 30% and 55%, more preferably between 35% and 53%, even more preferably between 40% and 50%, of the absorbent core length (L). This advantageously allows the miction point positioning to correspond to the central section, so that liquid distribution towards front and back may quickly start.

In these same embodiments, preferably, the central section (41) has a length (413) of between 2 and 25% or between 4 and 25%, more preferably between 5 and 20%, even more preferably between 6 and 15 %, most preferably between 7 and 12%, of the absorbent core length (L). When the central section is too short, the absorption time may increase and rewet, to some extent, also may increase. When the central section is too long, both absorption time and rewet may greatly increase. Advantageously the length of the channel (CL,40) taken along the longitudinal center line (LCL) is between 50 and 90%, preferably between 55 and 80%, more preferably between 60 and 75%, of the absorbent core length (L).

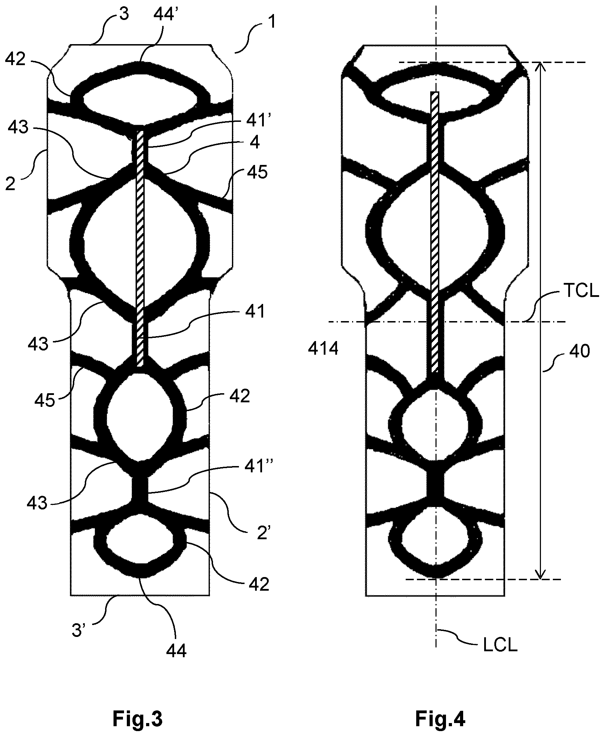

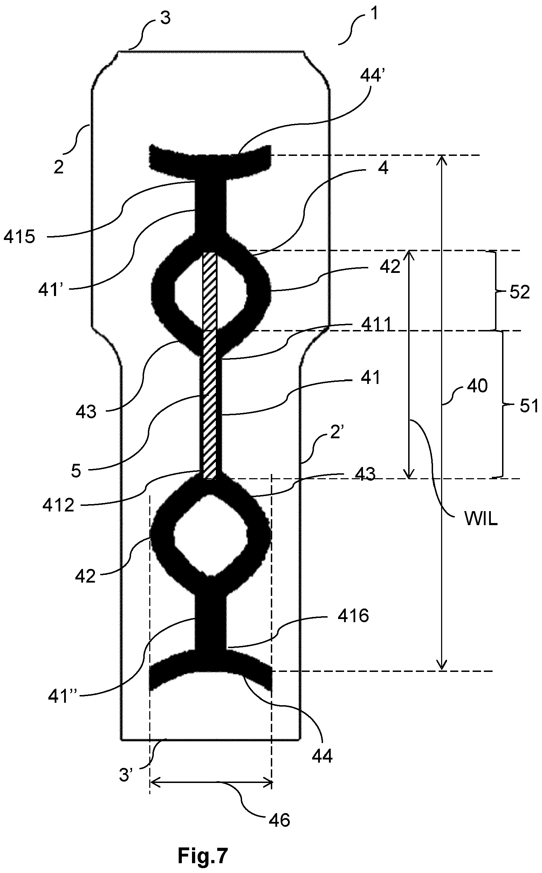

In alternative embodiments the channel (4) comprises at least 2 central sections, one (41) closest to or crossing the transverse center line (TCL) and at least another (41') remote from the transverse center line (TCL), 4, preferably 6, lateral sections (42), 6, preferably 8, diagonal sections (43) and 1, preferably 2, ending sections (44). Such pattern may provide a powerful draining system and/or comfort to the wearer. As examples, Figures 3 and 4 illustrate a channel (4) comprising 3 central sections (41, 41', 41"), 8 lateral sections (42), 12 diagonal sections (43) and two closing sections (44). As another example, Figure 5 illustrates a channel (4) comprising 3 central sections (41, 41', 41"), 6 lateral sections (42), 10 diagonal sections (43) and two ending sections (44), one of them being a closing section. As still other examples, Figures 6 and 7 illustrate each a channel (4) comprising 3 central sections (41, 41', 41"), 4 lateral sections (42), 8 diagonal sections (43) and two ending sections (44) which are not closing sections. The ending sections which are not closing sections may offer a better adhesion of the top and bottom core wrap within the channel.

In these alternative embodiments where the channel comprises at least 2 central sections, the central section (41) closest to or crossing the transverse center line (TCL), preferably the central section closest to or including the miction point, is preferably longitudinally centered on a point (414) which is at a distance from the front transverse edge along the longitudinal center line (LCL) of between 30% and 70%, more preferably between 40% and 65%, most preferably between 45% and 60%, of the absorbent core length (L). This advantageously allows the miction point positioning to correspond to the central section, so that liquid distribution towards front and back may quickly start and shorter acquisition times may be obtained.

In these alternative embodiments, preferably, the central section (41) closest to or crossing the transverse center line (TCL), preferably the central section closest to or including the miction point, has a length (413) of between 5 and 40%, more preferably between 5 and 25%, or between 10 and 30%, more preferably between 10% and 20%, or between 15 and 25% of the absorbent core length (L). When the central section is too short, the absorption time may increase and rewet, to some extent, also may increase. When the central section is too long, both absorption time and rewet may greatly increase.

In these alternative embodiments, the channel (4) may advantageously further comprise at least two diverging sections (45) connecting lateral sections (42) and/or diagonal sections (43) to the longitudinal edges (2,2') of the absorbent core (1). These diverging sections may further help the liquid distribution through the entire core and/or improve the comfort and fit. Preferably, however, the absorbent core is free of any diverging section connecting the channel to the front or back transverse edges to reduce the risk of leakage in these zones.

In these alternative embodiments, the length of the channel (CL,40) taken along the longitudinal center line (LCL) is preferably between 50 and 95%, more preferably between 60 and 90% or between 70 and 95%, still more preferably between 70 and 85% or between 80 and 95%, even more preferably between 83 and 93% of the absorbent core length (L).

In these alternative embodiments, the ratio of the channel surface area, i.e. the surface area substantially free of absorbent material (in black in figures 3-7), to the surface area of absorbent material (in white in figures 3-7) is preferably between 10 and 50%, more preferably between 15 and 40% or 20 and 50%, still more preferably between 20 and 30% or 30 and 40%. When this ratio is too low there might be not enough or not efficient enough fluid transport, and if the ratio is too high there might be a greater likelihood of leakage as liquid will flow faster to the edges of the core compared to the speed of acquisition into the absorbent material.

In accordance with the invention, the channel sections may be straight lines or may be curved lines. Preferably, the closing section is substantially curvilinear in shape, for example in the form of a U-bend with its convex side facing the absorbent core transverse edge at closest proximity, or is substantially linear in shape, for example forming a straight or triangular shape between the lateral sections end points. Alternatively, the ending section, when not a closing section, is preferably substantially curvilinear in shape with its concave side facing the absorbent core transverse edge at closest proximity. The lateral sections may be curved lines as visible in Figures 3-7, thereby creating with the diagonal sections, which may optionally be curved as well, a substantially oval, substantially almond-shaped, substantially elliptical or substantially circular channel shape surrounding or encircling absorbent material.

According to the invention, preferably, the absorbent core is free of any channel or channel section extending up to the front and back transverse edges of the absorbent core. This has been found advantageous in that it creates transversally extending zones of absorbent material acting as barriers to absorb liquid before it reaches the front and back end of the core, thereby avoiding leakage in those generally risky areas of the core.

Advantageously, the lateral sections (42) of the first longitudinal portion may be aligned on a first longitudinal axis parallel to the longitudinal center line (LCL), and the lateral sections (42) of the second longitudinal portion may be aligned on a second longitudinal axis parallel to the longitudinal center line (LCL). This may render the manufacturing process easier, ensuring the adhesion of the core wrap top and bottom layers along lines parallel to the manufacturing direction. Preferably the channel is symmetric about the longitudinal center line.

According to the invention, the one or more channels are formed by the adhesion of the top layer of the core wrap with the bottom layer of said core wrap, in zones substantially free of absorbent material. Such adhesion may advantageously provide core stability and for example reduce the risk of sagging. This adhesion may be provided by any means known in the art to bond core wrap layers together, for example with adhesive or mechanical bonding.

In an embodiment, the top and bottom core wrap layers are adhered by one or more adhesives. The adhesive may be either uniformly applied within the channels or it may be applied in zones of the channels such to form zones, preferably alternating zones, of different bonding strength between the core wrap layers. Ways to achieve stronger bonding strength in some zones may include using higher amounts of adhesive in said zones, applying greater mechanical pressure on said zones, or utilizing a different adhesive type.

In an embodiment, the top and bottom core wrap layers are adhered along the channels, at a plurality of discrete joining areas, preferably wherein said discrete joining areas are free of adhesive and typically comprise mechanical bonds. Advantageously, this allows for a permanent bonding of the top and bottom layers of the core wrap that limits the presence of additional hydrophobic substances such as adhesives therebetween whilst maximizing the unbonded spaces therebetween to enhance the fluid flow through the channel.

In an embodiment the bonding strength in some zones of the channels is less than in others, and the top core wrap layer and bottom core wrap layer may separate in said zones. This arrangement may allow the bonding in some zones to fail upon for example swelling of the SAP such to allow more volume to be available for expansion thereof (and prevent early saturation or non-optimal absorption), with typically the zones resisting such expansion providing integrity of the channels even in wet state.

The core wrap layers are preferably nonwoven webs. Advantageously, at least one of the top and bottom core wrap layers is an elastic nonwoven (e.g. containing an elastic material such as Vistamaxx resin from ExxonMobil, or other suitable polymers capable of imparting elasticity to a nonwoven web). An advantage of this embodiment is that the nonwoven web better and more easily wraps around the 3D insert during the manufacturing process.

According to the invention, the absorbent core comprises absorbent material selected from the group consisting of cellulose fibers, superabsorbent polymers and combinations thereof. The absorbent material may in particular comprise cellulosic fluff and/or fibrous web typically comprising airlaid fibers of the cellulosic kind. The superabsorbent polymers may typically be in the form of a plurality of discrete particles that may be distributed within the absorbent material or directly agglomerated in one or more pockets between at least two nonwoven webs. The absorbent core may include cellulose fibers and superabsorbent polymers in a proportion of 5-55 Wt% cellulose and 45-95 Wt% SAP.

In an embodiment, in addition to the channel having the sections as described above, the core further comprises one or more disconnected channels, an advantage being effective added local uniform fluid distribution.

In an embodiment, the width of the channels is constant throughout a continuous path of a channel. Alternatively, it may vary along a channel. The width of the channels may be between 3 and 20 mm, preferably between 5 and 15 mm, more preferably between 5 and 12 mm.

The cores herein may have various shapes. Preferably the width of the core in the region of the transverse center line is less than in at least one of the front or back portions. This region may be positioned in a crotch portion of the absorbent article such to provide better ergonomics and fit along the leg of a wearer. It is preferred that said cores are symmetric at least about the longitudinal axis thereof. Irrespective of the core geometry, it is understood herein that the same or similar channels as described herein may be interchangeably used.

In an embodiment, the absorbent core is a multi-layer core comprising at least a first and a second distinct core layers positioned one on top of the other. The first and/or the second core layer may comprise at least one channel according to the invention. Preferably they both comprise at least one channel according to the invention, wherein the shape and/or dimensions and/or positioning of said channel is substantially identical in both core layers.

Absorbent articles according to the present invention include disposable diapers or diaper pants; disposable incontinence diapers or diaper pants; sanitary pads, sanitary napkins and panty liners. They comprise a core sandwiched between a liquid permeable topsheet and a liquid impermeable backsheet. The backsheet may comprise a print or graphic viewable from the garment facing side of said article that substantially matches the shape and/or contour of the channel(s). This has the advantage to further accentuate the visual perception of the presence of such channel and its location in the absorbent article.

The absorbent articles may further comprise an acquisition distribution layer (ADL), also referred to herein as acquisition and distribution system, positioned between said topsheet and said core. The ADL may be positioned at a body-facing side of the absorbent core, between the topsheet and the absorbent core of the absorbent article, and more preferably in close proximity or even in good contact (most preferably in direct contact) with the body-facing side of the absorbent core. The use of an ADL in combination with the channels of the present invention may lead to an extremely good distribution of fluids from a discharge area to the entire absorbent core whilst attaining excellent perceived dryness performance.

The acquisition distribution system may be a single layer of spunbond and/or carded (e.g. carded thermobonded, in particular a carded web which has been subjected to thermal calendering) nonwoven. Alternatively, the acquisition distribution system may be multi-layered and comprise at least one spunbond layer and at least one meltblown layer typically the layers being nonwoven layers. Preferably, the acquisition distribution system is a nonwoven selected from the group consisting of: SM, SMS, SMMS, and combinations thereof.

In an embodiment, the acquisition distribution layer comprises a plurality of layers, wherein at least one of said layers, preferably each of said layers, consists of spunbond, meltblown and/or carded (e.g. thermocarded via calendering) nonwoven and wherein at least the layer most distal from the body-facing side of the absorbent core consists of spunbond and/or carded (e.g. thermocarded via calendering) nonwoven, preferably wherein both the layer most distal and the layer most proximal to the body-facing (also referred to herein as "skin-facing") side of the absorbent core consists of spunbond and/or carded (e.g. thermocarded via calendering) nonwoven.

In an embodiment, the acquisition distribution layer for use herein comprises synthetic fibers which are comprised at a level of greater than 80%wt by weight of said acquisition distribution layer. The acquisition distribution layer may have a basis weight of from 5 to 50 $g/m^2$, 10 to 50 $g/m^2$, preferably from 15 to 40 $g/m^2$, more preferably from 18 to 35 $g/m^2$, even more preferably from 20 to 30 $g/m^2$, most preferably from 21 to 25 $g/m^2$.

In an embodiment, the acquisition distribution system is the top layer of the core wrap and the absorbent article is free of additional layers, such as an acquisition distribution layer, so that the top layer of the core wrap is in direct contact with the topsheet. Said top layer of the core wrap may comprise a spunbond and/or carded, e.g. carded thermobonded by calendering, nonwoven layer comprising synthetic fibers, wherein preferably said synthetic fibers are comprised at a level of greater than 80%wt by weight of said layer, and/or wherein said top layer of the core wrap has a basis weight of from 5 to 50 $g/m^2$. Alternatively, said top layer of the core wrap may be multi-layered and comprise at least one spunbond layer and at least one meltblown layer typically the layers being nonwoven layers. Preferably, the multi-layered top layer of the core wrap acting as acquisition distribution system is a nonwoven selected from the group consisting of: SM, SMS, SMMS, and combinations thereof. Advantageously, by choosing core wraps as described, improved performance on rewet may be achieved even whilst eliminating the presence of further acquisition distribution layers, thus further introducing cost benefits.

## EXAMPLES

Examples 1 to 4:

Tests have been performed on diapers according to the invention to demonstrate the sequence in which the wetness indicator colour change occurs, thereby showing some of the benefits of the invention. 4 identical diapers with an absorbent core, a channel and a wetness indicator as illustrated in Figure 8 have been receiving different quantities of liquid in different timing patterns.

The diapers were of the size MINI type, the absorbent core length of around 300 mm, the channel length of about 220 mm and the wetness indicator length of about 150mm. The wetness indicator was coincident with the longitudinal center line (LCL) and superposed with the longitudinally extending channel central section, with absorbent material closer to the front transverse edge in a front substantially elliptical channel shape surrounding absorbent material (front ellipse, FE), and with absorbent material closer to the back transverse edge in a back substantially elliptical channel shape surrounding absorbent material (back ellipse, BE). The wetness indicator was of the colour changing type, commercially available from the company H.B. Fuller under reference FULL-CARE™ 9300. This is a hot-melt adhesive which was slot-coated on the internal surface of the liquid impermeable backsheet, i.e. on the side of the backsheet facing the absorbent core.

First the miction point (MP) was marked on each of the diapers. If we consider the total length of a diaper is x (in mm), the miction point is set at a distance of ( $\frac{x}{2} - 25$ ) towards the front of the diaper, on the longitudinal center line (LCL).

Each diaper was then, in turn, placed and maintained in cup shaped position in a bended apparatus (60) as illustrated in Figure 15, at room temperature.

This apparatus (60) comprises an arc-shaped transparent plate (61) in and along which a diaper can be placed and maintained in position, e.g. with clamps, with its full surface against the internal face of the plate. The width (610) of the arc-shaped plate is 21,5 cm. The apparatus (60) also comprises a horizontal rectangular transparent plate (62) which has a length (620) of 36 cm and a width (621) of 21,5 cm, and which joins the two sides of the arc-shaped plate at a height (611) taken along the axis of symmetry (X) of the arc shape equal to 20 cm. This horizontal plate (62) comprises a hole (63) in its center. The apparatus (60) further comprises a tube (64) of 30 mm diameter and of 22 cm length. It is generally inclined with an angle β of about 14° from verticality (X).

The apparatus is positioned with a camera under the apparatus to precisely record the wetness indicator colour change.

Each diaper was placed with the marked miction point at the center of the tube bottom end, and a coloured saline solution (0.9 %) at 38°C was poured in the tube.

The saline solution was prepared with 9 g NaCl dissolved in 991 g demineralised (or with adequate pureness) water (conductivity < 5 $\mu$S/cm). A food colorant free of common salt was added to the solution.

For the diaper of example 1, in a first step, 25 ml of coloured saline solution were poured in the tube. When these 25 ml were all absorbed and after a pause of 15 seconds, in a second step, 25 ml of coloured saline solution were poured again in the tube.

For the diaper of example 2, in a first step, 40 ml of coloured saline solution were poured in the tube. When these 40 ml were all absorbed and after a pause of 15 seconds, in a second step, 40 ml of coloured saline solution were poured again in the tube.

For the diaper of example 3, 40 ml of coloured saline solution were poured in the tube in a single step.

For the diaper of example 4, 75 ml of coloured saline solution were poured in the tube in a single step.

The time for the wetness indicator colour to start changing was recorded at different positions within the diaper, as seen from the garment-facing side of the article by the camera through the arc-shaped transparent plate. Recorded values are given in Table I.

TABLE I

| Wetness indicator time of reaction (start of colour change) [s] | | Within back ellipse | Within channel central section | Within front ellipse |
|---|---|---|---|---|
| Example 1 | 25 ml / 15 s / 25 ml | 15 | 7 | 21 |
| Example 2 | 40 ml / 15 s / 40 ml | 12 | 8 | 22 |
| Example 3 | 40 ml (single shot) | 14 | 5 | 19 |
| Example 4 | 75 ml (single shot) | 10 | 5 | 10 |

These results show that the overlapping portion of the wetness indicator, superposed with the longitudinally extending channel central section, quickly reacts, but that the exposed portion of the wetness indicator, superposed with absorbent material closer to the front of the diaper, which is the one mostly visible by the caregiver, reacts at a much later stage. This offers a close monitoring of sequence of wetness events, with a various steps colour change, the frontal part of the wetness indicator showing the last moment before changing the diaper.

**Claims**

1. An absorbent article having a wearer-facing side and a garment-facing side comprising a liquid permeable topsheet on the wearer-facing side, a liquid impermeable backsheet on the garment-facing side and an absorbent core (1) positioned in between said topsheet and said backsheet, said absorbent core (1) having a first and second longitudinal edge (2,2') and a front and back transverse edge (3,3'), said absorbent core (1) having a longitudinal center line (LCL) dividing the absorbent core (1) in a first longitudinal portion and a second longitudinal portion on either side of the longitudinal center line (LCL), said absorbent core (1) having a transverse center line (TCL) dividing the absorbent core (1) in a front portion and a back portion on either side of the transverse center line (TCL), thereby forming four quadrants, wherein the absorbent core (1) comprises absorbent material selected from the group consisting of cellulose fibers, superabsorbent polymers and combinations thereof, wherein said absorbent material is contained within at least one core wrap substrate enclosing said absorbent material, and wherein a top layer of said core wrap is adhered to a bottom layer of said core wrap to form one or more channels (4) substantially free of said absorbent material, wherein at least one channel (4) follows a substantially continuous path from any point of said channel to any other point of the same channel, and comprises at least one longitudinally extending channel central section (41) coincident with the longitudinal center line (LCL) and closest to or crossing the transverse center line (TCL), **characterised in that** the absorbent article further comprises a continuously extending wetness indicator (5), which as seen from the garment-facing side of the article, is coincident with the longitudinal center line (LCL) and comprises an overlapping portion (51) superposed with at least part of the longitudinally extending channel central section (41), and an exposed portion (52) superposed with absorbent material at least partially surrounded by channel sections (42,43) closer to the front transverse edge (3).

2. An absorbent article according to claim 1, wherein the wetness indicator (5) further comprises an exposed portion (53) superposed with absorbent material at least partially surrounded by channel sections (42,43) closer to the back transverse edge (3').

3. An absorbent article according to claim 1 or claim 2, wherein the wetness indicator (5) extends beyond the channel front extremity.

4. An absorbent article according to any of the preceding claims, wherein the wetness indicator (5) has a total length (WIL) between 30 and 70% of the absorbent core length (L)

5. An absorbent article according to any of the preceding claims, wherein the wetness indicator (5) provides an appearing signal, a disappearing signal, a colour change signal or a combination thereof.

6. An absorbent article according to any of the preceding claims, wherein the wetness indicator (5) is placed between the bottom side of the core wrap and the inner surface of the backsheet.

7. An absorbent article according to any of the preceding claims, wherein the wetness indicator (5) comprises a composition that changes appearance when contacted with body fluid, which is slot coated or printed on the side of the backsheet facing the absorbent core.

8. An absorbent article according to any of the preceding claims, wherein the wetness indicator (5) comprises a composition that changes appearance when contacted with body fluid and comprises at least one interruption zone (53) without wetness indicator composition having a length (54) parallel to the longitudinal center line (LCL) of less than 20% of the total length of the wetness indicator (WIL).

9. An absorbent article according to any of the preceding claims, wherein the opacity of the layers closer to the wearer-facing side of the article than the wetness indicator overlapping portion (51) is such as to allow to see the wetness indicator overlapping portion (51) through said layers, viewed from the wearer-facing side of the article.

10. An absorbent article according to any of the preceding claims, wherein the channel (4) additionally comprises at least:

    - one longitudinally extending lateral section (42) in each of the four quadrants,
    - four diagonally extending diagonal sections (43), diverging from the central section end points (411,412) towards the lateral sections in each quadrant and connecting said central (41) and lateral (42) sections, and
    - one transversally extending ending section (44,44'), in one of the front or back portions, closest to the absorbent core respective front or back transverse edge (3,3').

11. An absorbent article according to any of the preceding claims, wherein said channel (4) comprises a second transversally extending ending section (44,44'), in the other of the front or back portions, closest to the absorbent core respective front or back transverse edge (3,3').

12. An absorbent article according to any of the preceding claims, wherein the transversally extending ending section (44,44') is a closing section joining each of two lateral sections end points (421,422) closest to the absorbent core respective front or back transverse edge (3,3').

13. An absorbent article according to claim 12, wherein said channel (4) consists of one central section (41), four lateral sections (42), four diagonal sections (43) and one or two closing section(s) (44), and wherein the central section (41) has a length (413) of between 4 and 25% of the absorbent core length (L).

14. An absorbent article according to any of the claims 1 to 11, wherein said channel (4) comprises at least 2 central sections (41,41',41"), 4 lateral sections (42), 6 diagonal sections (43) and one or two ending sections (44), and wherein the central section (41) closest to or crossing the transverse center line (TCL) has a length (413) of between 5 and 40% of the absorbent core length (L).

**Patentansprüche**

1. Absorbierender Artikel, der eine dem Träger zugewandte Seite und eine dem Kleidungsstück zugewandte Seite aufweist, umfassend eine flüssigkeitsdurchlässige Oberschicht auf der dem Träger zugewandten Seite, eine flüssigkeitsundurchlässige Unterschicht auf der der Kleidung zugewandten Seite und einen absorbierenden Kern (1), der zwischen der Oberschicht und der Unterschicht angeordnet ist, wobei der absorbierende Kern (1) einen ersten und einen zweiten Längsrand (2, 2') sowie einen vorderen und einen hinteren Querrand (3, 3') aufweist, wobei der absorbierende Kern (1) eine Längsmittellinie (LCL) aufweist, die den absorbierenden Kern (1) auf beiden Seiten der Längsmittellinie (LCL) in einen ersten Längsabschnitt und einen zweiten Längsabschnitt unterteilt, wobei der absorbierende Kern (1) eine Quermittellinie (TCL) aufweist, die den absorbierenden Kern (1) auf jeder Seite der Quermittellinie (TCL) in einen vorderen Abschnitt und einen hinteren Abschnitt unterteilt, wodurch vier Quadranten gebildet werden, wobei der absorbierende Kern (1) absorbierendes Material umfasst, das ausgewählt ist aus der Gruppe, bestehend aus Zellulosefasern, superabsorbierenden Polymeren und Kombinationen davon, wobei das absorbierende Material in mindestens einem Kernumhüllungssubstrat enthalten ist, das das absorbierende Material umschließt, und wobei eine Obere Lage der Kernumhüllung an eine untere Lage der Kernumhüllung geklebt ist, um einen oder mehrere Kanäle (4) zu bilden, die im Wesentlichen frei von dem absorbierenden Material sind, wobei mindestens ein Kanal (4) einem im Wesentlichen kontinuierlichen Weg von jedem Punkt des Kanals zu jedem anderen Punkt desselben Kanals folgt und mindestens einen sich in Längsrichtung erstreckenden Kanalmittelabschnitt (41) umfasst, der mit der Längsmittellinie (LCL) zusammenfällt und der Quermittellinie (TCL) am nächsten ist oder diese kreuzt, **dadurch gekennzeichnet, dass** der absorbierende Artikel ferner einen sich kontinuierlich erstreckenden Nässeindikator (5) umfasst, der, von der dem Kleidungsstück zugewandten Seite des Artikels aus gesehen, mit der Längsmittellinie (LCL) zusammenfällt und einen überlappenden Abschnitt (51) umfasst, der mit mindestens einem Teil des sich in Längsrichtung erstreckenden mittleren Kanalabschnitts (41) überlagert ist, und einen freiliegenden Abschnitt (52) umfasst, der mit absorbierendem Material überlagert ist, das zumindest teilweise von Kanalabschnitten (42, 43) umgeben ist, die näher an dem vorderen Querrand (3) sind.

2. Absorbierender Artikel nach Anspruch 1, wobei der Nässeindikator (5) ferner einen freiliegenden Abschnitt (53) umfasst, der mit absorbierendem Material überlagert ist, das zumindest teilweise von Kanalabschnitten (42, 43) umgeben ist, die näher an dem hinteren Querrand (3') sind.

3. Absorbierender Artikel nach Anspruch 1 oder Anspruch 2, wobei sich der Nässeindikator (5) über das vordere Ende des Kanals hinaus erstreckt.

4. Absorbierender Artikel nach einem der vorherigen Ansprüche, wobei der Nässeindikator (5) eine Gesamtlänge (WIL) zwischen 30 und 70% der Länge (L) des absorbierenden Kerns aufweist.

5. Absorbierender Artikel nach einem der vorherigen Ansprüche, wobei der Nässeindikator (5) ein Erscheinungssignal, ein Verschwindensignal, ein Farbänderungssignal oder eine Kombination davon bereitstellt.

6. Absorbierender Artikel nach einem der vorherigen Ansprüche, wobei der Nässeindikator (5) zwischen der unteren Seite der Kernumhüllung und der Innenseite der Unterschicht platziert ist.

7. Absorbierender Artikel nach einem der vorherigen Ansprüche, wobei der Nässeindikator (5) eine Zusammensetzung umfasst, die ihr Aussehen bei Kontakt mit Körperflüssigkeit verändert und die auf der dem absorbierenden Kern zugewandten Seite der Rückschicht schlitzbeschichtet oder aufgedruckt ist.

8. Absorbierender Artikel nach einem der vorherigen Ansprüche, wobei der Nässeindikator (5) eine Zusammensetzung umfasst, die ihr Aussehen bei Kontakt mit Körperflüssigkeit verändert, und mindestens eine Unterbrechungszone (53) ohne Nässeindikator-Zusammensetzung mit einer Länge (54) parallel zur Längsmittellinie (LCL) von weniger als 20% der Gesamtlänge des Nässeindikators (WIL) umfasst.

9. Absorbierender Artikel nach einem der vorherigen Ansprüche, wobei die Opazität der Lagen, die näher an der dem Träger zugewandten Seite des Artikels sind als der Nässeindikator-Überlappungsabschnitt (51), derart ist, dass der Nässeindikator-Überlappungsabschnitt (51) von der dem Träger zugewandten Seite des Artikels aus gesehen durch die Lagen sichtbar ist.

10. Absorbierender Artikel nach einem der vorherigen Ansprüche, wobei der Kanal (4) zusätzlich mindestens Folgendes umfasst:

    - ein sich in Längsrichtung erstreckender seitlicher Abschnitt (42) in jedem der vier Quadranten,
    - vier sich diagonal erstreckende diagonale Abschnitte (43), die von den Endpunkten (411, 412) des mittleren Abschnitts zu den seitlichen Abschnitten in jedem Quadranten divergieren und den mittleren (41) und den seitlichen (42) Abschnitt verbinden, und

- einen sich quer erstreckenden Endabschnitt (44, 44') in einem von dem vorderen oder dem hinteren Abschnitt, der dem absorbierenden Kern bzw. einem vorderen oder einem hinteren Querrand (3, 3') am nächsten ist.

11. Absorbierender Artikel nach einem der vorherigen Ansprüche, wobei der Kanal (4) einen zweiten, sich quer erstreckenden Endabschnitt (44, 44') in dem anderen von dem vorderen oder hinteren Abschnitt umfasst, der dem absorbierenden Kern bzw. einem vorderen oder einem hinteren Querrand (3, 3') am nächsten ist.

12. Absorbierender Artikel nach einem der vorherigen Ansprüche, wobei der sich quer erstreckende Endabschnitt (44, 44') ein Verschlussabschnitt ist, der jeden der beiden Endpunkte (421, 422) der seitlichen Abschnitte verbindet, die dem absorbierenden Kern bzw. einem vorderen oder einem hinteren Querrand (3, 3') am nächsten sind.

13. Absorbierender Artikel nach Anspruch 12, wobei der Kanal (4) aus einem mittleren Abschnitt (41), vier seitlichen Abschnitten (42), vier diagonalen Abschnitten (43) und einem oder zwei Verschlussabschnitt(en) (44) besteht, und wobei der mittlere Abschnitt (41) eine Länge (413) zwischen 4 und 25 % der Länge (L) des absorbierenden Kerns aufweist.

14. Absorbierender Artikel nach einem der Ansprüche 1 bis 11, wobei der Kanal (4) mindestens 2 mittlere Abschnitte (41, 41', 41"), 4 seitliche Abschnitte (42), 6 diagonale Abschnitte (43) und einen oder zwei Endabschnitte (44) umfasst, und wobei der mittlere Abschnitt (41), der der transversalen Mittellinie (TCL) am nächsten ist oder diese kreuzt, eine Länge (413) zwischen 5 und 40% der Länge (L) des absorbierenden Kerns aufweist.

**Revendications**

1. Article absorbant ayant une face tournée vers la personne qui le porte et une face tournée vers les vêtements comprenant une couche supérieure perméable au liquide sur la face tournée vers la personne qui porte l'article, une couche arrière imperméable au liquide sur la face tournée vers les vêtements et un noyau absorbant (1) positionné entre ladite couche supérieure et ladite couche arrière, ledit noyau absorbant (1) ayant un premier et un second bords longitudinaux (2,2') et un bord transversal avant et arrière (3,3'), ledit noyau absorbant (1) ayant une ligne centrale longitudinale (LCL) qui divise le noyau absorbant (1) dans une première partie longitudinale et une seconde partie longitudinale de n'importe quel côté de la ligne centrale longitudinale (LCL), ledit noyau absorbant (1) ayant une ligne centrale transversale (TCL) qui divise le noyau absorbant (1) en une partie avant et une partie arrière de n'importe quel côté de la ligne centrale transversale (TCL), afin de former quatre quadrants, dans lequel le noyau absorbant (1) comprend un matériau absorbant choisi parmi le groupe consistant en des fibres de cellulose, des polymères super-absorbants et des combinaisons de ceux-ci, dans lequel ledit matériau absorbant est contenu dans au moins un substrat à gainage qui contient ledit matériau absorbant, et dans lequel une couche supérieure dudit gainage adhère sur une couche inférieure dudit gainage afin de former un ou plusieurs canaux (4) sensiblement exempts dudit matériau absorbant, dans lequel au moins un canal (4) suit un trajet sensiblement continu depuis n'importe quel point dudit canal jusqu'à n'importe quel autre point du même canal, et comprend au moins une section centrale de canal qui s'étend longitudinalement (41) coïncidente avec la ligne centrale longitudinale (LCL) et la plus proche de ou qui croise la ligne centrale transversale (TCL), **caractérisé en ce que** l'article absorbant comprend en outre un indicateur d'humidité qui s'étend en continu (5), qui, vu depuis la face tournée vers les vêtements de l'article, coïncide avec la ligne centrale longitudinale (LCL) et comprend une partie en chevauchement (51) superposée sur au moins une partie de la section centrale de canal qui s'étend longitudinalement (41), et une partie exposée (52) superposée sur le matériau absorbant au moins partiellement entourée par des sections de canal (42, 43) plus proches du bord transversal avant (3).

2. Article absorbant selon la revendication 1, dans lequel l'indicateur d'humidité (5) comprend en outre une partie exposée (53) superposée sur le matériau absorbant au moins partiellement entourée par des sections de canal (42, 43) plus proches du bord transversal arrière (3').

3. Article absorbant selon la revendication 1 ou 2, dans lequel l'indicateur d'humidité (5) s'étend au-delà de l'extrémité avant du canal.

4. Article absorbant selon l'une quelconque des revendications précédentes, dans lequel l'indicateur d'humidité (5) possède une longueur totale (WIL) comprise entre 30 et 70 % de la longueur du noyau absorbant (L).

5. Article absorbant selon l'une quelconque des revendications précédentes, dans lequel l'indicateur d'humidité (5) fournit un signal d'apparition, un signal de disparition, un signal de changement de couleur ou une combinaison de ceux-ci.

**6.** Article absorbant selon l'une quelconque des revendications précédentes, dans lequel l'indicateur d'humidité (5) est placé entre la face inférieure du gainage et la surface interne de la couche arrière.

**7.** Article absorbant selon l'une quelconque des revendications précédentes, dans lequel l'indicateur d'humidité (5) comprend une composition qui change d'apparence lorsqu'elle est mise en contact avec un fluide corporel, qui est recouverte de fentes ou imprimée côté couche arrière tournée vers le noyau absorbant.

**8.** Article absorbant selon l'une quelconque des revendications précédentes, dans lequel l'indicateur d'humidité (5) comprend une composition qui change d'apparence lorsqu'elle est mise en contact avec un fluide corporel et qui comprend au moins une zone d'interruption (53) sans composition d'indicateur d'humidité qui présente une longueur (54) parallèle à la ligne centrale longitudinale (LCL) inférieure à 20 % de la longueur totale de l'indicateur d'humidité (WIL).

**9.** Article absorbant selon l'une quelconque des revendications précédentes, dans lequel l'opacité des couches plus proches de la face tournée vers la personne qui porte l'article que la partie en chevauchement de l'indicateur d'humidité (51) est telle qu'elle permet de voir la partie en chevauchement de l'indicateur d'humidité (51) à travers lesdites couches, vue depuis la face tournée vers la personne qui porte l'article.

**10.** Article absorbant selon l'une quelconque des revendications précédentes, dans lequel le canal (4) comprend en outre au moins :

- une section latérale qui s'étend longitudinalement (42) dans chacun des quatre quadrants,
- quatre sections diagonales qui s'étendent en diagonale (43), qui divergent des points d'extrémité de la section centrale (411, 412) vers les sections latérales dans chaque quadrant et qui relient lesdites sections centrale (41) et latérale (42), et
- une section d'extrémité qui s'étend transversalement (44,44'), dans l'une des parties avant ou arrière, la plus proche du bord transversal avant ou arrière respectif du noyau absorbant (3,3').

**11.** Article absorbant selon l'une quelconque des revendications précédentes, dans lequel ledit canal (4) comprend une seconde section d'extrémité qui s'étend transversalement (44,44'), dans l'autre des parties avant ou arrière, la plus proche du bord transversal avant ou arrière respectif du noyau absorbant (3,3').

**12.** Article absorbant selon l'une quelconque des revendications précédentes, dans lequel la section d'extrémité qui s'étend transversalement (44,44') est une section de fermeture qui joint chacun des deux points d'extrémité des sections latérales (421,422) les plus proches du bord transversal avant ou arrière respectif du noyau absorbant (3,3').

**13.** Article absorbant selon la revendication 12, dans lequel ledit canal (4) se compose d'une section centrale (41), de quatre sections latérales (42), de quatre sections diagonales (43) et d'une ou deux section(s) de fermeture (44), et dans lequel la section centrale (41) possède une longueur (413) comprise entre 4 et 25 % de la longueur du noyau absorbant (L).

**14.** Article absorbant selon l'une quelconque des revendications 1 à 11, dans lequel ledit canal (4) comprend au moins 2 sections centrales (41,41',41"), 4 sections latérales (42), 6 sections diagonales (43) et une ou deux section(s) d'extrémité (44), et dans lequel la section centrale (41) la plus proche de ou qui croise la ligne centrale transversale (TCL) présente une longueur (413) comprise entre 5 et 40 % de la longueur du noyau absorbant (L).

**Fig.1**     **Fig.2**

**Fig.3**

**Fig.4**

**Fig.5**            **Fig.6**

**Fig.7**

**Fig.8**

**Fig.9**

**Fig.10**

**Fig.11**

**Fig.12**

**Fig.13**

**Fig.14**

**Fig.15**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 3342386 A1
- EP 20171374
- WO 2010078304 A1
- WO 2015095514 A2
- WO 2019125230 A1
- US 2017079857 A
- WO 2016196069 A1
- EP 3190216 A1